# EUROPÄISCHE PATENTSCHRIFT

(11) **EP 0 877 631 B1**
(45) Veröffentlichungstag und Bekanntmachung des Hinweises auf die Patenterteilung: **16.04.2003**
(21) Anmeldenummer: 97907031.5
(22) Anmeldetag: 23.01.1997
(51) Int. Cl.: A61K 49/00

(54) **KONJUGAT AUS ALBUMIN UND EINEM XANTHEN-FARBSTOFF ZUR UNTERSCHEIDUNG VON KRANKHAFTEM UND GESUNDEM GEWEBE**
CONJUGATE OF ALBUMIN AND A XANTHEN DYE FOR DIFFERENTIATING BETWEEN DISEASED AND HEALTHY TISSUES
CONJUGUE D'ALBUMINE AVEC UN COLORANT DU TYPE XANTHENE PERMETTANT DE DIFFERENCIER UN TISSU SAIN D'UN TISSU MALADE

(30) Priorität: 23.01.1996 DE 19602295
(43) Veröffentlichungstag der Anmeldung: 18.11.1998
(73) Patentinhaber: DEUTSCHES KREBSFORSCHUNGSZENTRUM STIFTUNG DES ÖFFENTLICHEN RECHTS, 69120 Heidelberg (DE)
(72) Erfinder: SINN, Hansjörg, D-69118 Wiesloch (DE); MAIER-BORST, Wolfgang, D-69221 Dossenheim (DE); STEHLE, Gerd, D-68305 Mannheim (DE); KAUS, Michael, D-69118 Heidelberg (DE); WUNDER, Andreas, D-69214 Eppelheim (DE); SCHRENK, Hans-Hermann, D-67728 Zeiskamm (DE); HOFF-BIEDERBECK, Dirk, 67259 Grossniedesheim (DE)
(74) Vertreter: Schüssler, Andrea, Dr.
(86) Internationale Anmeldenummer: DE9700166
(87) Internationale Veröffentlichungsnummer: WO97026920

(56) Entgegenhaltungen:
- US-A- 4 213 904
- "BIOCHEMICALIEN ORGANISCHE VERBINDINGEN VOOR RESEARCH EN DIAGNOSTICA" 1994 , SIGMA CHEMICAL COMPANY , NL XP002043421 siehe Seite 68 siehe Seite 344
- RUSHFELDT C ET AL: "Distribution of colon cancer cells permanently labeled by lectin-mediated endocytosis of a trap label." CANCER RES, 1. FEB 1993, VOL. 53, NO. 3, SEITEN 658-62, XP002043417
- DALESANDRO MR ET AL: "Temporal variations in the fine specificity of IgM anti-fluorescyl antibodies." IMMUNOL CELL BIOL, AUG 1991, VOL. 69, PART 4, SEITEN 243-51, XP002043418
- MOORE C H ET AL: "Synthesis of erythrosin isothiocyanate and its use as a phosphorescent depolarization probe for slow rotational mobility of membrane proteins" BIOCHEM. SOC. TRANS., 1979, VOL. 7, NO. 5, PAGE(S) 945-6, XP002043419
- SPEIRS A ET AL: "Segmental motion and rotational diffusion of the calcium-translocating adenosine triphosphatase of sarcoplasmic reticulum, measured by time-resolved phosphorescence depolarization" BIOCHEM. J., 1983, VOL. 213, NO. 1, PAGE(S) 67-74, XP002043420
- BIENIARZ C ET AL: "THIOLATE AND PHOSPHOROTHIOATE FUNCTIONALIZED FLUORESCEINS AND THEIR USE AS FLUORESCENT LABELS" BIOCONJUGATE CHEMISTRY, Bd. 5, Nr. 1, Seiten 31-39, XP000430383
- CHEMICAL ABSTRACTS, vol. 100, no. 23, 4.Juni 1984 Columbus, Ohio, US; abstract no. 190020, WATT R M ET AL: "Affinity labeling of antifluorescyl antibodies" XP002043422 & FLUORESCEIN HAPTEN: IMMUNOL. PROBE, 1984, PAGES 177-81, STATE UNIV. NEW YORK;UPSTATE MED. CENT.; SYRACUSE; NY; USA (US),
- YAN C ET AL: "Characterization and morphological analysis of protein-loaded poly(lactide-co-glycolide) microparticles prepared by water-in-oil-in-water emulsion technique" JOURNAL OF CONTROLLED RELEASE, Bd. 32, Nr. 3, 1994, Seiten 231-241, XP004037656

## Beschreibung

Die Erfindung betrifft Konjugate zur Unterscheidung von krankhaftem und gesundem Gewebe, Verfahren zur Herstellung solcher Konjugate sowie ihre Verwendung.

In der Behandlung von krankhaftem Gewebe ist die Entfernung desselben oft ein essentieller Schritt. Hierzu ist es notwendig, daß der operierende Arzt genau erkennt, wo krankhaftes Gewebe endet und gesundes Gewebe beginnt. Dies ist allerdings oft nicht möglich. Ausläufer des krankhaften Gewebes werden daher übersehen, die dann die Basis für die erneute Entstehung des krankhaften Gewebes darstellen.

Rushfeldt et al. (Cancer Res., Vol. 53, Nr. 3., S. 658-662, 1993) beschreiben eine Methode zum Markieren von Colon-Karzinomen mit Fluorescein unter Verwendung spezieller Lektin-ähnlicher Rezeptoren für die Endozytose.

"Biochemicalien Organische Verbindungen Voor Research en Diagnostica, 1994, Sigma, NL, S. 71" erwähnt ein Konjugat von humanem Serumalbumin mit Fluorescein.

Der vorliegenden Erfindung liegt somit die Aufgabe zugrunde, ein Mittel bereitzustellen, mit dem krankhaftes von gesundem Gewebe unterschieden werden kann.

Erfindungsgemäß wird dies durch die Gegenstände in den Patentansprüchen erreicht.

Gegenstand der vorliegenden Erfindung ist somit ein Konjugat aus einem Xanthen-Farbstoff, einem Linker und nativem humanem Serumalbumin, wobei der Linker Cyanurchlorid ist.

Vertreter der Xanthen-Farbstoffe sind Fluorescein, Aminofluorescein (AFI), Erythrosin, Aminoerythrosin (AEros), Eosin gelblich und Aminoeosin gelblich (AEs). Vorstehende Verbindungen können auch eine Photoaktivität aufweisen. Ein Beispiel dafür ist AEros. Typischerweise wird die photodynamische Aktivität bei einer Wellenlänge im Bereich von 500 bis 550 nm (AEros: 533 nm) angeregt.

In einem erfindungsgemäßen Konjugat können mehrere Xanthen-Farbstoffe vorliegen, die gleich oder verschieden voneinander sein können.

In einem erfindungsgemäßen Konjugat liegt humanes Serumalbumin (HSA) vor Dieses Protein bewirk eine Anreicherung des Konjugats in krankhaften Geweben, insbesondere in Tumorgewebe und in oberflächlich gelegenen kleineren Gefäßen, z.B. von Neovaskularisationen im Bereich der Cornea (Hornhaut des Auges).

Der Ausdruck "Linker" umfaßt Cyanurchlorid (Cy).

Vorstehende Komponenten eines erfindungemäßen Konjugats sind als Edukte angegeben. Im Konjugat liegen sie derivatisiert vor.

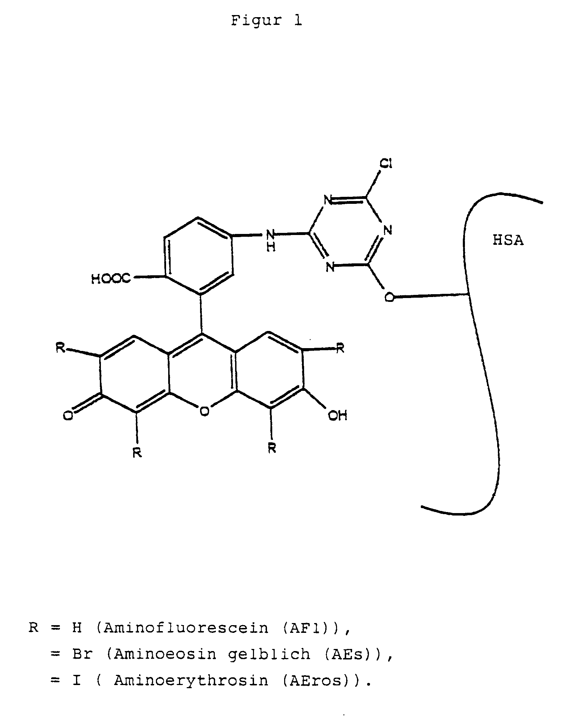

Bevorzugte erfindungsgemäße Konjugate sind in der Figur 1 angegeben.

Erfindungsgemäße Konjugate können nach üblichen Verfahren hergestellt werden, durch welche der Xanthen-Farbstoff, der Linker und das Serumalbumin miteinander, insbesondere kovalent, verbunden werden. Beispielhaft wird hierzu auf die Herstellung der Konjugate der Beispiele 1 bis 3 verwiesen.

Erfindungsgemäße Konjugate zeichnen sich durch eine erhöhte Halbwertszeit im Organismus aus. Desweiteren reichern sich erfindungsgemäße Konjugate in krankhaften Geweben an, insbesondere in Tumorgewebe und in oberflächlich gelegenen kleineren Gefäßen, z.B. von Neovaskularisationen im Bereich der Cornea. Durch Licht, z.B. einer UV-Lampe, wird die zur Fluoreszenz-fähige Verbindung angeregt, wodurch krankhafte Gewebe sichtbar gemacht werden, wohingegen gesundes Gewebe, in dem sich die erfindungsgemäßen Konjugate nicht anreichern, nicht sichtbar gemacht wird. Daher ist eine Abgrenzung von krankhaften Geweben gegenüber gesundem Gewebe sehr gut möglich.

Weiterhin zeichnen sich erfindungsgemäße Konjugate darin aus, daß bei ihnen eine photodynamische Aktivität bei einer Wellenlänge im Bereich von 500 bis 550 nm angeregt werden kann. Licht dieser Wellenlänge hat im Körper nur eine geringe Eindringtiefe. Somit eignen sich erfindungsgemäße Konjugate bestens zur Therapie von oberflächlich gelegenen, krankhaften Geweben, z.B. Neovaskularisationen und Ösophagustumoren.

### Kurze Beschreibung der Zeichnung:

- Figur 1:: zeigt die erfindungsgemäßen Konjugate AEs-Cy-HSA, AEros-Cy-HSA und AFI-Cy-HSA und
- Figur 2:: zeigt die Anreicherung von AFI-Cy-HSA in Tumorgewebe.

Die folgenden Beispiele erläutern die Erfindung.

### Beispiel 1 Herstellung des erfindungsgemäßen Konjugats AEros-Cy-HSA

AEros-Cy-HSA ist in Figur 1 dargestellt.

50 mg 5([4,6-Dichlortriazin-2-yl]amino)-fluorescein ( = AFI-Cy) wurden in 5 ml DMSO gelöst und durch Zugabe einer entsprechenden Menge von Jodsuccinimid in 2 ml DMSO und 1 ml 0,17M Bic (Natriumbicarbonat) zu AEros-Cy umgesetzt. Die Umsetzung war nach wenigen Sekunden beendet. Das so hergestellte AEros-Cy wurde zu 4g HSA, das in 20 ml Originallösung, 20 ml Bic und 20 ml DMSO gelöst war, unter ständigem Rühren langsam zugegeben. Die Lösung färbte sich tiefrot; sie blieb klar. Nach etwa 45 Minuten wurde die Proteinlösung mit 1 I dest. Wasser verdünnt und nachfolgend durch Ultrafiltration (YM 30, Amicon) gereinigt. Die analytische Reinheitskontrolle erfolgte mittels HPLC. Es wurde das erfindungsgemäße Konjugat AEros-Cy-HSA erhalten.

### Beispiel 2 Herstellung des erfindungsgemäßen Konjugats AEs-Cy-HSA

AEs-Cy-HSA ist in Figur 1 dargestellt.

Die Herstellung von AEs-Cy-HSA erfolgte analog dem in Beispiel 1 beschriebenen Verfahren, wobei anstelle von lodsuccinimid Bromsuccinimid eingesetzt wurde.

### Beispiel 3 Herstellung des erfindunsgemäßen Konjugats AFI-Cy-HSA

AFI-Cy-HSA ist in Figur 1 dargestellt.

45 mg AFI-Cy wurden in 4 ml DMSO gelöst und zu 4 g HSA, das in 20 ml Originallösung, 20 ml Bic und 20 ml DMSO gelöst war, unter ständigem Rühren langsam zugegeben. Die Lösung färbte sich während der AFI-Cy-Zugabe intensivgelb; sie blieb klar. Nach etwa 45 Minuten wurde die erhaltene Lösung mit 1 I dest. Wasser verdünnt und nachfolgend durch Ultrafiltration (YM 30, Amicon) gereinigt. Die Reinheitskontrolle erfolgte mittels HPLC. Es wurde AFI-Cy-HSA erhalten.

### Beispiel 4 Anreicherung von AFI-Cy-HSA in einem Tumor

Eine Ratte (240 g), die ein Walker-256-Karzinosarkom aufwies (Tumorgewicht 4,2% des Körpergewichts), erhielt eine intravenöse Injektion von AFI-Cy-HSA von Beispiel 4. Dieses wurde vorher radioaktiv markiert. Nach 24 Stunden wurde die Ratte getötet. Alle Organe und der Tumor wurden entfernt. Die Mengen des in den Organen und im Tumor befindlichen Konjugats wurde durch Messung der Radioaktivität bestimmt.

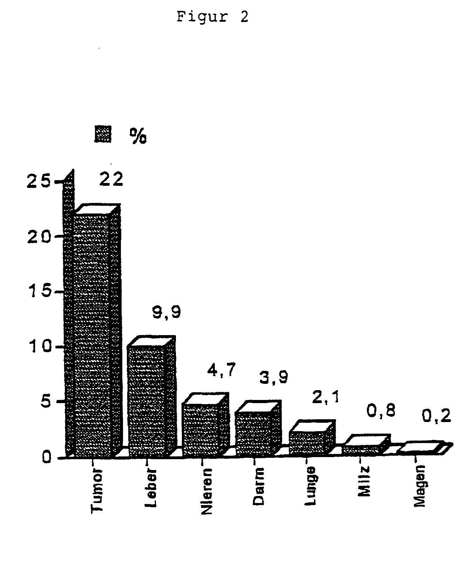

Wie aus Figur 2 zu sehen ist, reicherte sich das erfindungsgemäße Konjugat AFI-Cy-HSA im Tumor an.

## Patentansprüche

1. Konjugat aus einem Xanthen-Farbstoff, einem Linker und nativem humanem Serumalbumin, wobei der Linker Cyanurchlorid ist.

2. Konjugat nach Anspruch 1, **dadurch gekennzeichnet, daß** der Xanthen-Farbstoff Fluorescein, Aminofluorescein, Erythrosin, Aminoerythrosin, Eosin gelblich, Aminoeosin gelblich ist.

3. Konjugat nach Anspruch 1 oder 2, **dadurch gekennzeichnet, daß** der Xanthen-Farbstoff eine Photoaktivität aufweist.

4. Konjugat nach einem der Ansprüche 1 bis 3, **dadurch gekennzeichnet, daß** mehrere Xanthen-Farbstoffe vorliegen.

5. Verfahren zur Herstellung eines Konjugats nach einem der Ansprüche 1 bis 4, **dadurch gekennzeichnet, daß** der Xanthen-Farbstoff, Cyanurchlorid und natives humanes Serumalbumin kovalent verbunden werden.

6. Verwendung eines Konjugats nach einem der Ansprüche 1 bis 4 zur Herstellung eines Mittels zur Unterscheidung von krankhaftem und gesundem Gewebe.

## Claims

1. A conjugate comprising a xanthene dyestuff, a linker and native human serum albumin, the linker being cyanuric chloride.

2. The conjugate according to claim 1, **characterized in that** the xanthene dyestuff is fluorescein, aminofluorescein, erythrosin, aminoerythrosin, eosin yellowish, aminoeosin yellowish.

3. The conjugate according to claim 1 or 2, **characterized in that** the xanthene dyestuff has photodynamic activity.

4. The conjugate according to any of claims 1 to 3, **characterized in that** several xanthene dyestuffs are present.

5. A method of preparing a conjugate according to any of claims 1 to 4, **characterized in that** the xanthene dyestuff, cyanuric chloride and native human serum albumin are bonded covalently.

6. Use of a conjugate according to any of claims 1 to 4 for the production of a product for making a distinction between pathological and healthy tissues.

## Revendications

1. Conjugué d'un colorant du type xanthène, d'un lieur et de sérum-albumine humaine native, dans lequel le lieur est le chlorure de cyanuryle.

2. Conjugué suivant la revendication 1, **caractérisé en ce que** le colorant du type xanthène est la fluorescéine, l'amino-fluorescéine, l'érythrosine, l'amino-érythrosine, l'éosine tirant sur le jaune, l'amino-éosine tirant sur le jaune.

3. Conjugué suivant la revendication 1 ou 2, **caractérisé en ce que** le colorant du type xanthène possède une photoactivité.

4. Conjugué suivant l'une des revendications 1 à 3, **caractérisé en ce que** plusieurs colorants du type xanthène sont présents.

5. Procédé de production d'un conjugué suivant l'une des revendications 1 à 4, **caractérisé en ce que** l'on lie par covalence le colorant du type xanthène, du chlorure de cyanuryle et de la sérum-albumine humaine native.

6. Utilisation d'un conjugué suivant l'une des revendications 1 à 4 pour la préparation d'un agent servant à différencier un tissu malade d'un tissu sain.
